(19) Europäisches Patentamt / European Patent Office / Office européen des brevets

(11) **EP 3 782 971 B1**

(12) **EUROPÄISCHE PATENTSCHRIFT**

(45) Veröffentlichungstag und Bekanntmachung des Hinweises auf die Patenterteilung:
**19.07.2023   Patentblatt 2023/29**

(21) Anmeldenummer: **20190854.8**

(22) Anmeldetag: **13.08.2020**

(51) Internationale Patentklassifikation (IPC):
**C07C 2/10** (2006.01)        **C07C 7/04** (2006.01)
**C07C 11/02** (2006.01)

(52) Gemeinsame Patentklassifikation (CPC):
(C-Sets verfügbar)
**C07C 2/10; C07C 7/04;** C07C 2523/755    (Forts.)

(54) **VERFAHREN ZUR OLIGOMERISIERUNG VON OLEFINEN MIT OPTIMIERTER DESTILLATION**

METHOD FOR OLIGOMERIZATION OF OLEFINS WITH OPTIMIZED DISTILLATION

PROCÉDÉ D'OLIGOMÉRISATION D'OLÉFINES À DISTILLATION OPTIMISÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.08.2019   EP 19192737**

(43) Veröffentlichungstag der Anmeldung:
**24.02.2021   Patentblatt 2021/08**

(73) Patentinhaber: **Evonik Operations GmbH**
**45128 Essen (DE)**

(72) Erfinder:
• **Peitz, Stephan**
  **45739 Oer-Erkenschwick (DE)**
• **Stochniol, Guido**
  **45721 Haltern am See (DE)**
• **Rix, Armin Matthias**
  **45770 Marl (DE)**
• **Paul, Niklas**
  **45770 Marl (DE)**
• **Six, Tanita Valèrie**
  **44309 Dortmund (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**EP-A2- 1 457 475     WO-A1-2009/095411**

(52) Gemeinsame Patentklassifikation (CPC): (Forts.)

C-Sets
**C07C 2/10, C07C 11/02;**
**C07C 7/04, C07C 11/02**

**Beschreibung**

Gegenstand der Erfindung ist ein Verfahren zur Oligomerisierung von C2- bis C8-Olefinen in mindestens zwei Reaktionsstufen, bei dem das Reaktionsgemisch in der letzten Destillationskolonne so aufgetrennt wird, dass nur sehr geringe Mengen der Eduktolefine und der analogen Alkane im Sumpf der Destillationskolonne verbleiben.

Generell versteht man unter der Oligomerisierung die Reaktion von ungesättigten Kohlenwasserstoffen mit sich selbst, wobei entsprechend längerkettige Kohlenwasserstoffe, die so genannten Oligomere, entstehen. So lässt sich beispielsweise durch die Oligomerisierung von zwei Olefinen mit drei Kohlenstoffatomen ein Olefin mit sechs Kohlenstoffatomen (Hexen) aufbauen. Die Oligomerisierung von zwei Molekülen miteinander wird auch Dimerisierung genannt.

Die erhaltenen Oligomere sind Zwischenprodukte, die beispielsweise für die Herstellung von Aldehyden, Carbonsäuren und Alkoholen eingesetzt werden. Die Oligomerisierung von Olefinen wird großtechnisch entweder in homogener Phase an einem gelösten oder heterogen an einem festen Katalysator oder mit einem zweiphasigen Katalysatorsystem durchgeführt.

Verfahren zur Oligomerisierung von Olefinen sind im Stand der Technik hinlänglich bekannt und werden großindustriell eingesetzt. Die Produktionsmengen belaufen sich alleine in Deutschland auf mehrere tausend Kilotonnen pro Jahr. Um möglichst hohe Umsätze und einen möglichst kontinuierlichen Betrieb von Oligomerisierungsverfahren zu ermöglichen, weisen industrielle Anlagen meist nicht nur eine, sondern mindestens zwei hintereinandergeschaltete Reaktionsstufen auf, die jeweils mindestens einen Reaktor umfassen. Dadurch kann das

Oligomerisierungsverfahren selbst bei Ausfall einer Reaktionsstufe weiterbetrieben werden. Dokument EP 1 457 475 A2 beschreibt ein Verfahren zur Herstellung von Oligomeren von Alkenen mit 4 bis 8 Kohlenstoffatomen in n aufeinander folgenden adiabatisch betriebenen Reaktoren mit jeweils nachgeschalteten Destillationseinheiten, bei welchem der Sumpfaustrag oder die Sumpfausträge der Destillationskolonne(n) aus den jeweiligen Reaktionsstufen nicht zur letzten Destillationskolonne der letzten Reaktionsstufe rückgeführt werden.

Dokument WO 2009/095411 A1 beschreibt ebenfalls ein Oligomerisierungsverfahren das in mehreren hintereinander geschalteten Reaktoren mit nachgeschalteten Destillationskolonnen durchgeführt wird. Auch hier werden der Sumpfaustrag oder die Sumpfausträge der Destillationskolonne(n) aus den jeweiligen Reaktionsstufen nicht zur letzten Destillationskolonne der letzten Reaktionsstufe rückgeführt.

Eine Reaktionsstufe umfasst weiterhin mindestens eine Destillationskolonne, um die gebildeten Oligomere von den eingesetzten Olefinen abzutrennen. Dabei wird der Einsatzolefin-haltige Strom als Destillat über Kopf genommen und über den Sumpf das an Einsatzolefinen abgereicherte Oligomerisat abgeführt. Es ist bekannt, dass bei einer nicht ausreichenden Trennung der Eduktolefine von den gebildeten Oligomeren im Sumpf die Reinheit der Oligomere in den nachfolgenden Prozessschritten, beispielsweise bei der Abtrennung der Oligomere von hochsiedenden Nebenprodukten, nicht eingehalten werden kann und deshalb zu Problemen führen kann.

Die Aufgabe der vorliegenden Erfindung war die Bereitstellung eines Verfahrens zur Oligomerisierung von Olefinen, welches die vorgenannten Probleme nicht aufweist, aber für den Bau einer mehrstufigen Oligomerisierung möglichst geringe Investitionskosten, gerade im Bereich der Kolonnen, aufweist. Die Kosten steigen dabei mit jeder zusätzlichen Trennstufe. Die zugrundeliegende Aufgabe der vorliegenden Erfindung konnte mit dem Verfahren zur Oligomerisierung gemäß Anspruch 1 gelöst werden. Bevorzugte Ausgestaltungen sind in den Unteransprüchen angegeben.

Das erfindungsgemäße Verfahren ist ein Verfahren zur Oligomerisierung von C2- bis C8-Olefinen, vorzugsweise C3- bis C6-Olefinen, weiterhin bevorzugt C3- bis C5-Olefinen und besonders bevorzugt C4-Olefinen in mindestens zwei hintereinandergeschalteten Reaktionsstufen, die jeweils mindestens einen Reaktor und mindestens eine Destillationskolonne umfassen, wobei ein Einsatzgemisch, welches zumindest die C2- bis C8-Olefine als Eduktolefine und einen Anteil von > 10 Gew.-% an deren analogen Alkanen und vorzugsweise bis zu 50 Gew.-% an deren analogen Alkanen enthält, in dem mindestens einen Reaktor unter Verwendung eines heterogenen Katalysators einer Oligomerisierung bei einem Eduktolefinumsatz von 60 bis 95%, vorzugsweise 70 bis 93%, besonders bevorzugt 80 bis 92% unterworfen wird und das aus dem mindestens einen Reaktor erhaltene Reaktionsgemisch in der mindestens einen Destillationskolonne destilliert wird, um die gebildeten Oligomere vom restlichen Reaktionsgemisch, welches zumindest die nicht umgesetzten Eduktolefine und die Alkane enthält und welches das Destillat der Destillationskolonne bildet, abzutrennen, wobei das in der mindestens einen Destillationskolonne gebildete Destillat zumindest teilweise zu dem oder den Reaktoren der gleichen oder vorherigen Reaktionsstufe geführt wird, dadurch gekennzeichnet, dass

der Sumpfaustrag oder die Sumpfausträge der Destillationskolonne(n) aus den jeweiligen Reaktionsstufen zur letzten Destillationskolonne der letzten Reaktionsstufe geführt werden und die Konzentration der Eduktolefine und/oder die Konzentration der Alkane im Sumpf der letzten Destillationskolonne < 200 Gew.-ppm, vorzugsweise < 150 Gew.-ppm, besonders bevorzugt < 100 Gew.-ppm beträgt, die Konzentration der Eduktolefine und/oder die Konzentration der Alkane hingegen in dem Sumpf oder in den Sümpfen der vorhergehenden Destillationskolonne(n) > 200 Gew.-ppm beträgt.

In dem Sumpf oder in den Sümpfen der vorhergehenden Destillationskolonne(n) kann die Konzentration der Eduktolefine und/oder die Konzentration der Alkane maximal 2 Gew.-% sein.

Der Begriff "Reaktionsstufe" meint im Sinne der vorliegenden Erfindung einen Anlagenabschnitt, der einen oder mehrere Reaktor(en) und eine oder mehrere dem Reaktor nachfolgende Destillationskolonne(n) umfasst. In einer bevorzugten Ausführungsform ist nur eine Destillationskolonne pro Reaktionsstufe vorhanden. In den Destillationskolonnen werden insbesondere die erzeugten Oligomere von dem restlichen Ausgangsstrom aus dem Reaktor, welcher beispielsweise Alkane und nicht umgesetzte Olefine umfasst, getrennt. Die Oligomere sind höhersiedend als die nicht umgesetzten Olefine und weitere im Ausgangsstrom aus dem Reaktor befindliche Stoffe und werden deshalb im Sumpf der Destillationskolonne angereichert, während die leichter siedenden nicht umgesetzten Olefine und ggf. im Einsatzstrom vorhandene analoge Alkane über Kopf der Destillationskolonne gehen und im Destillat angereichert werden. Übliche verfahrenstechnische Aggregate, die in den Reaktionsstufen eingebaut sein können, wie beispielsweise Vorwärmer für den Feed, Wärmetauscher oder ähnliches, werden hier nicht separat aufgeführt, sondern sind dem Fachmann geläufig.

Die üblicherweise eingesetzten Einsatzgemische für die Oligomerisierung bestehen zu einem signifikanten Anteil aus inerten Alkanen, hier mehr als 10 Gew.-% und vorzugsweise bis zu 50 Gew.-%. Der Massenstrom an Alkanen bleibt über die gesamte mehrstufigen Oligomerisierung aufgrund ihrer Inertheit konstant. Ein wirtschaftliches Betreiben einer Oligomerisierung ist nur mit Umsätzen an Olefinen von <100% möglich, da aufgrund der Mehrstufigkeit der Anlage die Olefinkonzentration im Zulauf der jeweils folgenden Stufe immer kleiner wird, während der Massenstrom an Alkanen gleich bleibt, was sich deutlich verlangsamend auf die Kinetik der Oligomerisierung auswirkt. Insofern ist gemäß dem vorliegenden Verfahren vorgesehen, dass der Eduktolefinumsatz auf einen Bereich von 60 bis 95%, vorzugsweise 70 bis 93%, besonders bevorzugt 80 bis 92% begrenzt wird.

Um aufgrund der negativen Entwicklung der Kinetik der Reaktion von Stufe zu Stufe dennoch einen ausreichend hohen Umsatz an Olefinen über das gesamte Verfahren zu erzielen, erfolgt ein zumindest teilweiser Recycle des Destillats der Destillationskolonne in den Reaktorzulauf. Die zumindest teilweise Rückführung des Destillats zu einem vorherigen Reaktor wird durchgeführt, um den verbleibenden Butenen ausreichend Verweilzeit für die Reaktion zu ermöglichen. Aus dem Rücklauf ergibt sich ein bestimmtes Verhältnis von Recycle (= zurückgeführtes Destillat) zu Frischfeed, welches auch Recycle-zu-Feed-Verhältnis genannt wird. Das Recycle-zu-Frischfeed-Verhältnis beträgt im vorliegenden Verfahren für jede der vorhandenen Reaktionsstufe vorzugsweise zwischen 0,1 und 5, besonders bevorzugt zwischen 0,1 und 3.

Es wurde überraschenderweise herausgefunden, dass eine nahezu Eduktolefin-freie Sumpffraktion nur in der letzten Destillationskolonne einer mindestens zwei Stufen umfassenden Oligomerisierung notwendig ist. Dadurch kann auch bei bestehenden Anlagen Energie gespart werden, da nicht mehr bei allen Destillationskolonnen alle Eduktolefine aus dem Sumpf abgetrennt werden müssen. Neu zu bauende Destillationskolonnen können bis auf die letzte Destillationskolonne kleiner gebaut werden, da die Trennleistung im Sumpf nicht bei einer vollständigen Abtrennung der Eduktkomponenten liegen muss. Stattdessen kann in dem Sumpf oder den Sümpfen der oder den vorhergehenden Destillationskolonnen zur Abtrennung der Oligomere von den Eduktolefinen eine Eduktolefinkonzentration von > 200 Gew.-ppm, vorzugsweise bis maximal 2 Gew.-% vorliegen. Diese Sumpfausträge werden zur letzten Destillationskolonne der letzten Reaktionsstufe geführt und dort weiter aufgereinigt. Dabei kann der Sumpfaustrag/Sumpfausträge über einen separaten Zulaufstutzen in die letzte Kolonne gefahren werden oder aber mit dem Austrag des letzten Reaktors gemischt zusammen in die Kolonne gefahren werden. Die Konzentration der Eduktolefine und/oder die Konzentration der Alkane im Sumpf der letzten Destillationskolonne beträgt < 200 Gew.-ppm, vorzugsweise < 150 Gew.-ppm, besonders bevorzugt < 100 Gew.-ppm.

In einer bevorzugten Ausführungsform der vorliegenden Erfindung kann außerdem die Konzentration der Oligomere im Destillat der letzten Destillationskolonne der letzten Reaktionsstufe < 100 Gew.-ppm, vorzugsweise < 80 Gew.-ppm, besonders bevorzugt < 50 Gew.-ppm betragen, während in dem oder den Destillaten aus der oder den vorhergehenden Destillationskolonnen eine Konzentration der Oligomere von > 200 Gew.-ppm und vorzugsweise maximal 2 Gew.-% vorliegt. Das in der mindestens einen Destillationskolonne gebildete Destillat wird zumindest teilweise zu dem oder den Reaktoren der gleichen oder vorherigen Reaktionsstufe, vorzugsweise der gleichen Reaktionsstufe zurückgeführt.

Das erfindungsgemäße Verfahren umfasst mindestens zwei Reaktionsstufen. In einer bevorzugten Ausführungsform umfasst das Verfahren zur Oligomerisierung maximal fünf Reaktionsstufen. Insbesondere bevorzugt ist eine Verfahrensführung mit drei oder vier Reaktionsstufen. Jede dieser Reaktionsstufen umfasst unabhängig voneinander einen oder mehreren Reaktoren und eine oder mehrere nachfolgende Destillationskolonnen, um die gebildeten Oligomere von dem restlichen Ausgangsstrom aus dem Reaktor abzutrennen. Es ist aber auch denkbar, dass eine der Reaktionsstufen mehrere Reaktoren umfasst, während in einer vorherigen oder nachfolgenden Reaktionsstufe nur ein Reaktor vorhanden ist.

Die Oligomerisierung von Olefinen ist eine exotherme Reaktion, also eine Reaktion, bei der Wärme freigesetzt wird. Um die Oligomerisierungstemperatur in einem gewünschten Bereich zu halten, können die Reaktoren unter Verwendung eines Kühlmediums gekühlt werden, um einen Großteil (mehr als 60%) der oder die gesamte freigewordene Wärme abzuführen. Dies entspricht einer isothermen Betriebsweise. Um die freigewordene Wärme für nachfolgende Prozesse zu nutzen, kann auf eine Kühlung teilweise oder vollständig verzichtet werden. Werden der oder die Reaktoren nicht aktiv gekühlt, spricht man von einer adiabatischen Betriebsweise. Die bei der Oligomerisierung freiwerdende Wärme wird durch den Austrag des Produktstroms aus dem Reaktor abgeführt und in der Destillationskolonne insofern genutzt wird. In der nachfolgenden Destillationskolonne wird dann weniger Energie zur Verdampfung benötigt und die Destillation kann damit energiesparender durchgeführt werden.

Der oder die Reaktoren in den jeweiligen Reaktionsstufen des erfindungsgemäßen Verfahrens können isotherm oder adiabat betrieben werden. In einer bevorzugten Ausführungsform wird bzw. werden nur der oder die Reaktoren der letzten Reaktionsstufe adiabat betrieben, während alle anderen Reaktoren der vorherigen Reaktionsstufe(n) aktiv gekühlt werden. Dabei kann ein dem Fachmann bekanntes Kühlmedium, beispielsweise Kühlwasser, eingesetzt werden. In einer bevorzugten Ausführungsform sollte der Temperaturanstieg im Reaktor trotz Kühlens 5 K nicht überschreiten. Dies entspricht einer isothermen Betriebsweise der Reaktoren. Bezogen auf eine Kühlleistung von 100% für den oder die Reaktor(en) in der ersten Reaktionsstufe, beträgt die Kühlleistung in dem oder den Reaktor(en) der nachfolgenden Reaktionsstufen weniger als 100%, aber außer in der letzten Reaktionsstufe nicht 0%.

In einer ganz bevorzugten Ausführungsform, bei Vorhandensein von drei Reaktionsstufen, beträgt die Kühlleistung für den oder die Reaktor(en) der ersten Reaktionsstufe 100% und für den oder die Reaktor(en) der zweiten Reaktionsstufe 10 bis 60%, wobei der Reaktor der dritten und letzten Reaktionsstufe adiabatisch betrieben wird. In einer weiteren ganz bevorzugten Ausführungsform, bei Vorhandensein von vier Reaktionsstufen, beträgt die Kühlleistung für den oder die Reaktor(en) der ersten Reaktionsstufe 100%, für den oder die Reaktor(en) der zweiten Reaktionsstufe 40 bis 60% und für den oder die Reaktor(en) der dritten Reaktionsstufe 10 bis 30%, wobei der Reaktor der vierten und letzten Reaktionsstufe adiabatisch betrieben wird.

Die bei der Kühlung in den der adiabat betriebenen vorhergehenden Reaktionsstufen durch das Kühlmedium aufgenommene Wärme kann in einer bevorzugten Ausführungsform genutzt werden, um einen oder mehrere der Zulaufströme, bevorzugt alle Zulaufströme zu den einzelnen Reaktionsstufen aufzuwärmen, vorzugsweise auf einen Temperatur T > 50 °C. Dies kann in dem Fachmann bekannter Weise durchgeführt werden, insbesondere durch die Verwendung eines Wärmetauschers. So kann die bei der Reaktion entstehende und über das Kühlmedium bei der Kühlung aufgenommene Wärme noch für das weitere Verfahren verwendet werden, was aus wirtschaftlicher und ökologischer Sicht vorteilhaft ist.

In den einzelnen Reaktionsstufen wird der jeweilige Zulaufstrom aus dem Einsatzgemisch erfindungsgemäß in dem mindestens einen Reaktor oligomerisiert und das erhaltene Produktgemisch jeweils zu einer Destillationskolonne geleitet, bei der die Eduktolefine als Destillat über Kopf von dem restlichen Produktgemisch abgetrennt werden. Je nach Reaktionsstufe kann das Destillat dann zumindest teilweise als Zulaufstrom zur jeweils nächsten Reaktionsstufe geleitet und optional teilweise zu dem oder zu den Reaktoren der gleichen oder einer vorherigen Reaktionsstufe recycliert werden. In der letzten Reaktionsstufe, also der zweiten, dritten, vierten, fünften oder nachfolgenden Reaktionsstufe, kann das Destillat zudem zumindest teilweise aus dem Verfahren ausgeschleust werden. Wird das Destillat der letzten Destillationskolonne der letzten Reaktionsstufe aus dem hier offenbarten Verfahren ausgeschleust, kann dies als Syntheserohstoff für weitere Verfahren (z. B. Hydroformylierung, C-Quelle für Lichtbogen bei der Acetylenherstellung), als Verbrennungsgas oder nach Vollhydrierung zu den Alkanen als Treibgas, als Kochgas o. ä. dienen.

Die Bedingungen der Destillation, also beispielsweise Temperatur und Druck, sind üblicherweise durch den Aufbau (Höhe Kolonne, Anzahl der Böden, Art der Böden bzw. Packung, Abstände usw.) festgelegt. Während des Betriebs können die Trenneigenschaften der Destillation noch über die Temperaturverteilung und/oder die Wärmezufuhr in der Kolonne und den Rücklauf im Destillat gesteuert werden. Ebenso kann die Trenneigenschaft durch die Veränderung des Drucks in einem gewissen Rahmen eingestellt werden. Die genauen Einstellungen lassen sich daher nicht übergeordnet und unabhängig von dem Aufbau der Destillationskolonne definieren, was dem Fachmann jedoch bekannt ist.

Das Einsatzgemisch für das erfindungsgemäße Verfahren enthält zumindest die C2- bis C8-Olefine, bevorzugt C3- bis C6-Olefine, weiterhin bevorzugt C3- bis C5-Olefine, besonders bevorzugt C4-Olefine und einen Anteil von > 10 Gew.-% an deren analogen Alkanen und vorzugsweise bis zu 50 Gew.-% an deren analogen Alkanen. Geeignete Olefine sind unter anderem $\alpha$-Olefine, n-Olefine und Cycloalkene, vorzugsweise n-Olefine. In einer bevorzugten Ausführungsform handelt es sich bei dem Olefin um n-Buten. Das analoge Alkan ist in diesem Fall Butan bzw. Isobutan.

Die Olefine werden üblicherweise nicht in reiner Form als Edukte eingesetzt, sondern in technisch verfügbaren Mischungen. Der in dieser Erfindung zusätzlich verwendete Begriff Einsatzgemisch ist deshalb so zu verstehend, dass er jegliche Art von Gemischen betrifft, die die entsprechenden zu oligomerisierenden Olefine in einer Menge enthalten, die es ermöglicht, die Oligomerisierung wirtschaftlich durchzuführen. Bevorzugt enthalten die erfindungsgemäß verwendeten Einsatzgemische praktisch keine weiteren ungesättigten Verbindungen und mehrfach ungesättigte Verbindungen wie Diene oder Acetylenderivate. Vorzugsweise werden Einsatzgemische eingesetzt, die weniger als 5 Gew.-%, insbesondere weniger als 2 Gew.-% an verzweigten Olefinen bezogen auf den Olefinanteil enthalten.

Propylen wird großtechnisch durch Spaltung von Naphtha hergestellt und ist eine Grundchemikalie, die leicht verfügbar ist. C5-Olefine sind in Leichtbenzinfraktionen aus Raffinerien oder Crackern enthalten. Technische Gemische, die lineare C4-Olefine enthalten, sind Leichtbenzinfraktionen aus Raffinerien, C4-Fraktionen aus FC- oder Steamcrackern, Gemische aus Fischer-Tropsch-Synthesen, Gemische aus der Dehydrierung von Butanen und durch Metathese oder aus anderen technischen Prozessen entstandene Gemische. Beispielsweise können für das erfindungsgemäße Verfahren geeignete Gemische linearer Butene aus der C4-Fraktion eines Steamcrackers gewonnen werden. Dabei wird im ersten Schritt Butadien entfernt. Dies geschieht entweder durch Extraktion(sdestillation) des Butadiens oder dessen Selektivhydrierung. In beiden Fällen wird ein praktisch butadienfreier C4-Schnitt erhalten, das sogenannte Raffinat I. Im zweiten Schritt wird Isobuten aus dem $C_4$-Strom entfernt, z. B. durch Herstellung von MTBE durch Umsetzung mit Methanol. Der jetzt isobutenfreie und butadienfreie C4-Schnitt, das sogenannte Raffinat II, enthält die linearen Butene und gegebenenfalls Butane. Wird daraus auch noch zumindest ein Teil des enthaltenen 1-Butens abgetrennt, erhält man das sogenannte Raffinat III.

In einer bevorzugten Ausführungsform werden im erfindungsgemäßen Verfahren C4-olefinhaltige Stoffströme als Einsatzgemisch zugeführt. Geeignete Olefingemische sind insbesondere das Raffinat II und das Raffinat III.

Mit n-Butenen als Eduktolefinen ergibt sich das folgende besonders bevorzugte Verfahren:

Verfahren zur Oligomerisierung von n-Butenen in mindestens zwei hintereinandergeschalteten Reaktionsstufen, die jeweils mindestens einen Reaktor und mindestens eine Destillationskolonne umfassen, wobei

ein Einsatzgemisch, welches zumindest die n-Butene als Eduktolefine und einen Anteil von > 10 Gew.-% an C4-Alkanen und vorzugsweise bis zu 50 Gew.-% an C4-Alkanen enthält, in dem mindestens einen Reaktor unter Verwendung eines heterogenen Katalysators einer Oligomerisierung bei einem Eduktolefinumsatz von 60 bis 95%, vorzugsweise 70 bis 93%, besonders bevorzugt 80 bis 92% unterworfen wird und das aus dem mindestens einen Reaktor erhaltene Reaktionsgemisch in der mindestens einen Destillationskolonne destilliert wird, um die gebildeten Buten-Oligomere (Octene und höhere Oligomere) vom restlichen Reaktionsgemisch, welches zumindest nicht umgesetzte -Butene und die C4-Alkane enthält und welches das Destillat der Destillationskolonne bildet, abzutrennen, wobei das in der mindestens einen Destillationskolonne gebildete Destillat zumindest teilweise zu dem oder den Reaktoren der gleichen oder vorherigen Reaktionsstufe geführt wird, dadurch gekennzeichnet, dass

der Sumpfaustrag oder die Sumpfausträge der Destillationskolonne(n) aus den jeweiligen Reaktionsstufen zur letzten Destillationskolonne der letzten Reaktionsstufe geführt werden und die Konzentration der Butene und/oder die Konzentration der weiteren C4-Verbindungen im Sumpf der letzten Destillationskolonne der letzten Reaktionsstufe < 200 Gew.-ppm, vorzugsweise < 150 Gew.-ppm, besonders bevorzugt < 100 Gew.-ppm beträgt, die Konzentration der Butene und/ oder die Konzentration der weiteren C4-Verbindungen hingegen in dem Sumpf oder in den Sümpfen der vorhergehenden Destillationskolonne(n) > 200 Gew.-ppm beträgt.

In dem Sumpf oder in den Sümpfen der vorhergehenden Destillationskolonne(n) kann die Konzentration der Butene und/oder die Konzentration der weiteren C4-Verbindungen maximal 2 Gew.-% sein.

Als Reaktor für die jeweiligen Reaktionsstufen können alle dem Fachmann bekannten Reaktoren eingesetzt werden, die für die Oligomerisierung geeignet sind, bspw. Rohrreaktoren, Rohrbündelreaktoren, Settler-Riser-Reaktoren oder Slurry-Reaktoren. Bevorzugt sind Rohrreaktoren und/oder Rohrbündelreaktoren. Sofern eine Reaktionsstufe mehrere Reaktoren aufweist, können die Reaktoren gleich oder verschieden voneinander sein. Die Reaktoren in einer Reaktionsstufe können auch in ihrem Aufbau oder ihrer Ausgestaltung variieren. Der erste Reaktor in einer Reaktionsstufe kann beispielsweise ein größeres Volumen aufweisen als der nachfolgende Reaktor in der gleichen Reaktionsstufe. Ebenso ist es möglich, dass die Reaktoren in den einzelnen Reaktionsstufen gleich oder verschieden voneinander sein. Auch dabei ist es möglich, dass die Reaktoren in den einzelnen Reaktionsstufen in ihrem Aufbau oder ihrer Ausgestaltung unterschiedlich sind. Der Reaktor in der ersten Reaktionsstufe kann beispielsweise ein größeres Volumen aufweisen als ein oder alle Reaktoren in den nachfolgenden Reaktionsstufen.

Der eine oder die Reaktoren der einzelnen Reaktionsstufen enthalten jeweils einen heterogenen Oligomerisierungskatalysator zur Durchführung der Oligomerisierung. Der verwendete Oligomerisierungskatalysator liegt dabei insbesondere in Form eines Granulats, eines Extrudats oder in Tablettenform vor.

Die (heterogenen) Oligomerisierungskatalysatoren kann eine Nickelverbindung, vorzugsweise Nickeloxid, auf einem Alumosilicat-Trägermaterial umfassen. Besonders bevorzugt ist es, dass die im erfindungsgemäßen Verfahren eingesetzten Katalysatoren weniger als 0,5 Gew.-%, bevorzugt weniger als 0,1 Gew.-%, besonders bevorzugt weniger als 0,01 Gew.-% Titandioxid und Zirkoniumdioxid bezogen auf die Gesamtzusammensetzung des Oligomerisierungskatalysators enthalten. Das Trägermaterial kann ein amorphes, mesoporöses Alumosilicat, ein kristallines, mikroporöses Alumosilicat oder eine Alumosilicat, welches amorphe und kristalline Phasen aufweist sein. Mit "amorph" im Sinne der vorliegenden Erfindung ist die Eigenschaft eines Feststoffes gemeint, die daraus folgt, dass der Feststoff im Gegensatz zu kristallinen Feststoffen keine Kristallstruktur, d. h. keine Fernordnung, aufweist.

Erfindungsgemäß bevorzugt weist der Oligomerisierungskatalysator eine Zusammensetzung von 15 bis 40 Gew.-%, vorzugsweise 15 bis 30 Gew.-% NiO, 5 bis 30 Gew.-% $Al_2O_3$, 55 bis 80 Gew.-% $SiO_z$ und 0,01 bis 2,5 Gew.-

%, vorzugsweise 0,05 bis 2 Gew.-% eines Alkalimetalloxids, vorzugsweise Natriumoxid, auf. Die Angaben beziehen sich auf eine Gesamtzusammensetzung von 100 Gew.-%. Der Oligomerisierungskatalysator ist im Wesentlichen frei von Titandioxid und Zirkoniumdioxid, insbesondere enthält der Oligomerisierungskatalysator weniger als 0,5 Gew.-%, bevorzugt weniger als 0,1 Gew.-%, besonders bevorzugt weniger als 0,01 Gew.-% Titandioxid und Zirkoniumdioxid in seiner Gesamtzusammensetzung.

Der Oligomerisierungskatalysator weist vorzugsweise eine spezifische Oberfläche (berechnet nach BET) von 150 bis 700 $m^2/g$, weiterhin bevorzugt von 190 bis 600 $m^2/g$, besonders bevorzugt von 220 bis 550 $m^2/g$ aufweisen. Die BET-Oberfläche wird mittels Stickstoff-Physisorption gemäß DIN-ISO 9277 (Stand: 2014-01) gemessen.

Die in den einzelnen Reaktoren in den Reaktionsstufen vorhandenen Oligomerisierungskatalysatoren können jeweils unabhängig voneinander aus den vorgenannten Substanzen ausgewählt werden. Die einzelnen Oligomerisierungskatalysatoren in den Reaktoren sind dabei nicht immer exakt identisch, sondern unterscheiden sich in der Zusammensetzung voneinander, möglicherweise auch nur in geringem Umfang. Dies liegt auch daran, dass selbst wenn zum Zeitpunkt der ersten Inbetriebnahme des erfindungsgemäßen Verfahrens jeder Reaktor eine völlig identische Katalysatorzusammensetzung enthält, sich diese Zusammensetzung während des Betriebs mit der Zeit durch verschiedenste Effekte im Laufe der Jahre (Regenerierter Katalysator verhält sich anders als neu hergestellte Katalysatoren, Abrieb während des Betriebes, unterschiedlich schnelle Alterung und/oder Vergiftung, etc.) ändern.

Die Herstellung eines Oligomerisierungskatalysators kann nach den bekannten Verfahren des Imprägnierens, wobei das Trägermaterial mit einer Lösung einer Übergangsmetallverbindung, insbesondere eine Nickelverbindung, beaufschlagt und danach kalziniert wird, oder Co-Fällung, bei der die gesamte Katalysatorzusammensetzung aus einer einzigen, zumeist wässrigen Lösung ausgefällt wird, erfolgen. Der Oligomerisierungskatalysator kann auch nach anderen, dem Fachmann geläufigen, Verfahren hergestellt werden.

Die Oligomerisierung kann in jeder der vorhandenen Reaktionsstufen bei einer Temperatur im Bereich von 50 bis 200 °C, vorzugsweise 60 bis 180 °C, bevorzugt im Bereich von 60 bis 130°C durchgeführt wird. Der Druck kann jeder der vorhandenen Reaktionsstufen von 10 bis 70 bar, bevorzugt von 20 bis 55 bar betragen. In einer bevorzugten Ausführungsform der vorliegenden Erfindung wird die Oligomerisierung in jeder Reaktionsstufe in flüssiger Phase durchgeführt. Sofern die Oligomerisierung in flüssiger Phase erfolgen soll, müssen die Parameter Druck und Temperatur hierfür so gewählt werden, dass das Einsatzgemisch (die eingesetzten Olefine oder Olefingemische) in flüssiger Phase vorliegt.

Die gewichtbasierten Raumgeschwindigkeiten (Reaktandmasse pro Katalysatormasse pro Zeit; weight hourly space velocity (WHSV)) befinden sich im Bereich zwischen 1 g Reaktand pro g Katalysator und pro h (= 1 $h^{-1}$) und 190 $h^{-1}$, vorzugsweise zwischen 2 $h^{-1}$ und 35 $h^{-1}$, besonders bevorzugt zwischen 3 $h^{-1}$ und 25 $h^{-1}$.

Insbesondere bei Verwendung eines Katalysators, welcher eine Nickelverbindung, vorzugsweise Nickeloxid, auf einem Alumosilicat-Trägermaterial umfasst, beträgt der Grad der Dimerisierung (auch "prozentuale Selektivität bezogen auf die Dimerisierung" genannt) nach der Oligomerisierung bezogen auf das umgesetzte Edukt mindestens 60 %, weiter bevorzugt mindestens 75 %, besonders bevorzugt mindestens 80%.

Die Linearität eines Oligomerisierungsprodukts bzw. von den entstandenen Dimeren wird durch den ISO-Index beschrieben und stellt einen Wert für die mittlere Anzahl an Methylverzweigungen im Dimer dar. So tragen (für Buten als Edukt) z.B. n-Oktene mit 0, Methylheptene mit 1 und Dimethylhexene mit 2 zum ISO-Index einer C8-Fraktion bei. Je niedriger der ISO-Index ist, umso linearer sind die Moleküle in der jeweiligen Fraktion aufgebaut. Der ISO-Index errechnet sich nach folgender allgemeiner Formel, wobei sich der Anteil der einzelnen Dimerfraktionen auf die Gesamt-dimer-Fraktion bezieht:

$$\frac{\text{(einfach verzweigte Dimere (Gew.-\%)} + 2 \times \text{zweifach verzweigte Dimere (Gew.-\%))}}{100}$$

Demnach besitzt ein Dimerengemisch mit einem ISO-Index von 1,0 im Mittel genau eine Methylverzweigung pro Dimerenmolekül.

Der ISO-Index des Produkts aus dem erfindungsgemäßen Oligomerisierungsverfahren beträgt vorzugsweise 0,8 bis 1,2, besonders bevorzugt 0,8 bis 1,15.

Die nach dem erfindungsgemäßen Verfahren hergestellten Oligomere werden unter anderem zur Herstellung von Aldehyden, Alkoholen und Carbonsäuren genutzt. So ergibt beispielsweise das Dimerisat aus linearen Butenen durch Hydroformylierung ein Nonanalgemisch. Dieses liefert entweder durch Oxidation die entsprechenden Carbonsäuren oder durch Hydrierung ein C9-Alkoholgemisch. Das $C_9$-Säuregemisch kann zur Herstellung von Schmiermitteln oder Sikkative verwendet werden. Das C9-Alkoholgemisch ist Vorstufe für die Herstellung von Weichmachern, insbesondere von Di-Nonyl-phthalaten oder DINCH, sowie DINCD.

**Patentansprüche**

1.  Verfahren zur Oligomerisierung von C2- bis C8-Olefinen in mindestens zwei hintereinandergeschalteten Reaktionsstufen, die jeweils mindestens einen Reaktor und mindestens eine Destillationskolonne umfassen, wobei

    ein Einsatzgemisch, welches zumindest die C2- bis C8-Olefine als Eduktolefine und einen Anteil von > 10 Gew.-% an deren analogen Alkanen enthält, in dem mindestens einen Reaktor unter Verwendung eines heterogenen Katalysators einer Oligomerisierung bei einem Eduktolefinumsatz von 60 bis 95%, vorzugsweise 70 bis 93%, besonders bevorzugt 80 bis 92% unterworfen wird und das aus dem mindestens einen Reaktor erhaltene Reaktionsgemisch in der mindestens einen Destillationskolonne destilliert wird, um die gebildeten Oligomere vom restlichen Reaktionsgemisch, welches zumindest die nicht umgesetzten Eduktolefine und die Alkane enthält und welches das Destillat der Destillationskolonne bildet, abzutrennen, wobei das in der mindestens einen Destillationskolonne gebildete Destillat zumindest teilweise zu dem oder den Reaktoren der gleichen oder vorherigen Reaktionsstufe geführt wird, **dadurch gekennzeichnet, dass**
    der Sumpfaustrag oder die Sumpfausträge der Destillationskolonne(n) aus den jeweiligen Reaktionsstufen zur letzten Destillationskolonne der letzten Reaktionsstufe geführt werden und die Konzentration der Eduktolefine und/oder die Konzentration der Alkane im Sumpf der letzten Destillationskolonne < 200 Gew.-ppm beträgt, die Konzentration der Eduktolefine und/oder die Konzentration der Alkane hingegen in dem Sumpf oder in den Sümpfen der vorhergehenden Destillationskolonne(n) > 200 Gew.-ppm beträgt.

2.  Verfahren zur Oligomerisierung nach Anspruch 1, wobei die Konzentration der Eduktolefine und/oder die Konzentration der Alkane im Sumpf der letzten Destillationskolonne < 150 Gew.-ppm beträgt.

3.  Verfahren zur Oligomerisierung nach Anspruch 1, wobei die Konzentration der Eduktolefine und/oder die Konzentration der Alkane im Sumpf der letzten Destillationskolonne < 100 Gew.-ppm beträgt.

4.  Verfahren zur Oligomerisierung nach einem der Ansprüche 1 bis 3, wobei in den Reaktoren der einzelnen Reaktionsstufen ein Oligomerisierungskatalysator eingesetzt wird, welcher eine Nickelverbindung auf einem Alumosilicat-Trägermaterial umfasst.

5.  Verfahren nach Anspruch 4, wobei der Katalysator weniger als 0,5 Gew.-% Titandioxid und Zirkoniumdioxid in seiner Gesamtzusammensetzung enthält.

6.  Verfahren zur Oligomerisierung nach Anspruch 4 oder 5, wobei die Oligomerisierungskatalysatoren in den Reaktoren der einzelnen Reaktionsstufen eine Zusammensetzung von 15 bis 40 Gew.-% NiO, 5 bis 30 Gew.-% $Al_2O_3$, 55 bis 80 Gew.-% SiOz und 0,01 bis 2,5 Gew.-% eines Alkalimetalloxids aufweist.

7.  Verfahren zur Oligomerisierung nach einem der Ansprüche 1 bis 6, wobei bezogen auf eine Kühlleistung von 100% für den oder die Reaktor(en) in der ersten Reaktionsstufe die Kühlleistung in dem oder den (Reaktor(en) der nachfolgenden Reaktionsstufen weniger als 100%, aber nur in der letzten Reaktionsstufe 0% beträgt.

8.  Verfahren zur Oligomerisierung nach einem der Ansprüche 1 bis 7, wobei die Oligomerisierung in jeder der vorhandenen Reaktionsstufen bei einer Temperatur im Bereich von 50 bis 200 °C durchgeführt wird.

9.  Verfahren zur Oligomerisierung nach einem der Ansprüche 1 bis 8, wobei der Druck bei der Oligomerisierung jeder der vorhandenen Reaktionsstufen von 10 bis 70 bar beträgt.

10. Verfahren zur Oligomerisierung nach einem der Ansprüche 1 bis 9, wobei das Verfahren ein Verfahren zur Oligomerisierung von C3- bis C6-Olefinen ist.

11. Verfahren zur Oligomerisierung nach einem der Ansprüche 1 bis 9, wobei das Verfahren ein Verfahren zur Oligomerisierung von C3- bis C5-Olefinen ist.

12. Verfahren zur Oligomerisierung nach einem der Ansprüche 1 bis 9, wobei das Verfahren ein Verfahren zur Oligomerisierung von C4-Olefinen ist.

13. Verfahren zur Oligomerisierung nach einem der Ansprüche 1 bis 12, wobei das Recycle-zu-Frischfeed-Verhältnis für jede der vorhandenen Reaktionsstufe zwischen 0,1 und 5, vorzugsweise zwischen 0,1 und 3 liegt.

**14.** Verfahren nach einem der Ansprühe 1 bis 13, wobei das Einsatzgemisch bis zu 50 Gew.-% an Alkanen enthält.

**Claims**

**1.** Process for oligomerization of C2- to C8-olefins in at least two serially connected reaction stages, each of which comprise at least one reactor and at least one distillation column, wherein

an input mixture containing at least the C2- to C8-olefins as reactant olefins and a proportion of > 10% by weight of their analogous alkanes is subjected to oligomerization in the at least one reactor using a heterogeneous catalyst with a reactant olefin conversion of 60 to 95%, preferably 70 to 93%, particularly preferably 80 to 92%, and the reaction mixture obtained from the at least one reactor is distilled in the at least one distillation column to separate the formed oligomers from the residual reaction mixture containing at least the unconverted reactant olefins and the alkanes and forming the distillate from the distillation column, wherein the distillate formed in the at least one distillation column is at least partially passed to the reactor(s) of the same or preceding reaction stage, **characterized in that**
the bottom output or the bottom outputs from the distillation column(s) are passed from the respective reaction stages to the last distillation column of the last reaction stage and the concentration of the reactant olefins and/or the concentration of the alkanes in the bottom of the last distillation column is < 200 ppmw while in the bottom or in the bottoms of the preceding distillation column(s) the concentration of the reactant olefins and/or the concentration of the alkanes is > 200 ppmw by contrast.

**2.** Process for oligomerization according to Claim 1, wherein the concentration of the reactant olefins and/or the concentration of the alkanes in the bottom of the last distillation column is < 150 ppmw.

**3.** Process for oligomerization according to Claim 1, wherein the concentration of the reactant olefins and/or the concentration of the alkanes in the bottom of the last distillation column is < 100 ppmw.

**4.** Process for oligomerization according to any of Claims 1 to 3, wherein the reactors of the individual reaction stages employ an oligomerization catalyst which comprises a nickel compound on an aluminosilicate support material.

**5.** Process according to Claim 4, wherein the catalyst contains less than 0.5% by weight of titanium dioxide and zirconium dioxide in its overall composition.

**6.** Process for oligomerization according to Claim 4 or 5, wherein the oligomerization catalysts in the reactors of the individual reaction stages has a composition of 15% to 40% by weight of NiO, 5% to 30% by weight of $Al_2O_3$, 55% to 80% by weight of $SiO_2$ and 0.01% to 2.5% by weight of an alkali metal oxide.

**7.** Process for oligomerization according to any of Claims 1 to 6, wherein based on a cooling power of 100% for the reactor(s) in the first reaction stage, the cooling power in the reactor(s) of the subsequent reaction stages is less than 100%, but 0% only in the last reaction stage.

**8.** Process for oligomerization according to any of Claims 1 to 7, wherein the oligomerization in each of the reaction stages present is carried out at a temperature in the range from 50°C to 200°C.

**9.** Process for oligomerization according to any of Claims 1 to 8, wherein the pressure in the oligomerization of each of the reaction stages present is 10 to 70 bar.

**10.** Process for oligomerization according to any of Claims 1 to 9, wherein the process is a process for oligomerization of C3- to C6-olefins.

**11.** Process for oligomerization according to any of Claims 1 to 9, wherein the process is a process for oligomerization of C3- to C5-olefins.

**12.** Process for oligomerization according to any of Claims 1 to 9, wherein the process is a process for oligomerization of C4-olefins.

**13.** Process for oligomerization according to any of Claims 1 to 12, wherein the recycle-to-fresh feed ratio for each of the reaction stages present is between 0.1 and 5, preferably between 0.1 and 3.

**14.** Process according to any of Claims 1 to 13, wherein the input mixture contains up to 50% by weight of alkanes.

**Revendications**

**1.** Procédé d'oligomérisation d'oléfines en C2-C8 dans au moins deux étages de réaction consécutifs, qui comprennent à chaque fois au moins un réacteur et au moins une colonne de distillation,

un mélange d'utilisation, qui contient au moins les oléfines en C2-C8 en tant qu'oléfines de départ et une proportion > 10% en poids de leurs alcanes analogues, étant soumis dans ledit au moins un réacteur avec utilisation d'un catalyseur hétérogène à une oligomérisation à une conversion des oléfines de départ de 60 à 95%, de préférence de 70 à 93%, de manière particulièrement préférée de 80 à 92% et le mélange réactionnel obtenu à partir dudit au moins un réacteur étant distillé dans ladite au moins une colonne de distillation pour séparer les oligomères formés du mélange réactionnel résiduel, qui contient au moins les oléfines de départ non transformées et les alcanes et qui forme le distillat de la colonne de distillation, le distillat formé dans ladite au moins une colonne de distillation étant guidé au moins partiellement vers le(s) réacteur(s) du même étage de réaction ou de l'étage de réaction précédent, **caractérisé en ce que**
le(s) produit(s) évacué(s) du fond de la/des colonne(s) de distillation est/sont guidé(s) à partir des étages de réaction respectifs vers la dernière colonne de distillation du dernier étage de réaction et la concentration en oléfines de départ et/ou la concentration en alcanes dans le fond de la dernière colonne de distillation est/sont < 200 ppm en poids, la concentration en oléfines de départ et/ou la concentration en alcanes dans le(s) fond(s) de la/des colonne(s) de distillation précédente(s) est/sont par contre > 200 ppm en poids.

**2.** Procédé d'oligomérisation selon la revendication 1, la concentration en oléfines de départ et/ou la concentration en alcanes dans le fond de la dernière colonne de distillation étant < 150 ppm en poids.

**3.** Procédé d'oligomérisation selon la revendication 1, la concentration en oléfines de départ et/ou la concentration en alcanes dans le fond de la dernière colonne de distillation étant < 100 ppm en poids.

**4.** Procédé d'oligomérisation selon l'une quelconque des revendications 1 à 3, un catalyseur d'oligomérisation, qui comprend un composé de nickel sur un matériau support d'aluminosilicate, étant utilisé dans les réacteurs des différents étages de réaction.

**5.** Procédé selon la revendication 4, le catalyseur contenant moins de 0,5% en poids de dioxyde de titane et de dioxyde de zirconium dans sa composition totale.

**6.** Procédé d'oligomérisation selon la revendication 4 ou 5, les catalyseurs d'oligomérisation dans les réacteurs des différents étages de réaction présentant une composition de 15 à 40% en poids de NiO, 5 à 30% en poids de $Al_2O_3$, 55 à 80% en poids de $SiO_2$ et 0,01 à 2,5% en poids d'un oxyde de métal alcalin.

**7.** Procédé d'oligomérisation selon l'une quelconque des revendications 1 à 6, la puissance de refroidissement dans le(s) réacteur(s) des étages de réaction consécutifs, par rapport à une puissance de refroidissement de 100% pour le(s) réacteur(s) dans le premier étage de réaction, étant inférieure à 100%, mais ne valant 0% que dans le dernier étage de réaction.

**8.** Procédé d'oligomérisation selon l'une quelconque des revendications 1 à 7, l'oligomérisation dans chacun des étages de réaction présents étant réalisée à une température dans la plage de 50 à 200°C.

**9.** Procédé d'oligomérisation selon l'une quelconque des revendications 1 à 8, la pression lors de l'oligomérisation dans chacun des étages de réaction présents étant de 10 à 70 bars.

**10.** Procédé d'oligomérisation selon l'une quelconque des revendications 1 à 9, le procédé étant un procédé d'oligomérisation d'oléfines en C3-C6.

**11.** Procédé d'oligomérisation selon l'une quelconque des revendications 1 à 9, le procédé étant un procédé d'oligomérisation d'oléfines en C3-C5.

**12.** Procédé d'oligomérisation selon l'une quelconque des revendications 1 à 9, le procédé étant un procédé d'oligomérisation d'oléfines en C4.

**13.** Procédé d'oligomérisation selon l'une quelconque des revendications 1 à 12, le rapport de l'alimentation en produit recyclé à produit frais pour chacun des étages de réaction présents étant situé entre 0,1 et 5, de préférence entre 0,1 et 3.

**14.** Procédé selon l'une quelconque des revendications 1 à 13, le mélange d'utilisation contenant jusqu'à 50% en poids d'alcanes.

**IN DER BESCHREIBUNG AUFGEFÜHRTE DOKUMENTE**

**In der Beschreibung aufgeführte Patentdokumente**

- EP 1457475 A2
- WO 2009095411 A1